# EUROPEAN PATENT APPLICATION

(11) **EP 4 817 614 A1**
(43) Date of publication of application: **07.10.2026**
(21) Application number: 24898244.9
(22) Date of filing: 29.11.2024
(51) Int. Cl.: A61K 31/606, A61P 37/00, A61P 17/06, A61K 31/196, A61P 39/06, A23L 33/10

(54) **COMPOSITION AND METHOD FOR TREATING AUTOIMMUNE DISEASES**

(30) Priority: 30.11.2023 KR 20230170944; 05.09.2024 KR 20240120574; 18.10.2024 KR 20240143429
(71) Applicant: GNT Pharma Co., Ltd., Yongin-si, Gyeonggi-do 17096 (KR)
(72) Inventor: LEE, Jin Hwan, Hwaseong-si, Gyeonggi-do 18505 (KR); CHOI, Dong Joon, Seoul 08096 (KR); WON, So Jung, Hwaseong-si, Gyeonggi-do 18505 (KR); CHOI, Eui Ju, Seoul 06297 (KR); YOUN, Hwa Young, Seoul 08010 (KR); GWAG, Byoung Joo, Yongin-si, Gyeonggi-do 16951 (KR)
(74) Representative: Maiwald GmbH
(86) International application number: PCT/KR2024/019390
(87) International publication number: WO 2025/116652

(57) **Abstract**

The present invention relates to a pharmaceutical composition and method for treating or preventing autoimmune diseases in a patient or subject in need thereof, the pharmaceutical composition comprising: a 5-benzylaminosalicylic acid compound represented by Chemical Formula (I) or a pharmaceutically acceptable salt thereof, and, for example, a pharmaceutically acceptable carrier suitable for oral, intraperitoneal, or intravenous administration. The composition comprising the 5-benzylaminosalicylic acid compound of Chemical Formula (I) or a pharmaceutically acceptable salt thereof can be used for the treatment of autoimmune diseases.

## Description

### TECHNICAL FIELD

The present application claims priority based on Korean application No. 10-2023-0170944 filed November 30, 2023, Korean application No. 10-2024-0120574 filed September 5, 2024, and Korean application No. 10-2024-0143429 filed October 18, 2024, the entire disclosures in the specification and drawings of which are incorporated herein by reference.

The present invention relates to a pharmaceutical composition and method for treating or preventing autoimmune diseases, comprising a 5-benzylaminosalicylic acid compound represented by Chemical Formula (I) or a pharmaceutically acceptable salt thereof.

### BACKGROUND ART

Autoimmune diseases are conditions in which the body attacks its own healthy endogenous tissues due to a malfunction of the body's immune system. To protect the body from foreign or dangerous substances, the immune system must be able to identify them. These substances include bacteria, viruses, parasites (e.g., worms), some cancer cells, and even transplanted organs and tissues. These substances include molecules that the immune system can identify and that can stimulate a series of immune responses. These molecules are called antigens. Antigens can be included inside or on the surface of cells (e.g., bacteria or cancer cells), or they can be parts of viruses. Some antigens, such as pollen or food molecules, exist on their own.

When specific lymphocytes (B cells and T cells) encounter an antigen, they learn how to attack it, defending the body against potentially harmful antigens. One of the body's primary immunological defenses against antigens is the production of antibodies by B cells. Antibodies bind tightly to specific antigens, either tagging them for attack or neutralizing them directly. The body produces a wide range of antibodies. Each antibody possesses distinct antigen-specificity. When the immune system is exposed to the same antigen again, it remembers it, allowing it to attack much more effectively.

Antigens are also present in the cells of the patient's or individual's own tissues. However, generally, the immune system does not react to antigens in the patient's or individual's own tissues, but only to antigens of external or dangerous substances. However, sometimes the immune system malfunctions, interpreting the body's own tissues as foreign substances and producing antibodies (called autoantibodies) that target and attack specific cells or tissues of the body. Nevertheless, since autoantibodies produced in small amounts do not cause autoimmune diseases, the presence of autoantibodies in the blood does not necessarily indicate the presence of an autoimmune disease.

The exact cause of autoimmune diseases is unknown. Furthermore, the cause may vary depending on the specific type of autoimmune disease the patient has. Although the precise cause of autoimmune diseases is unknown, T-cell immune regulation is a common and important factor in their development. There are various types of T cells, the most well-known being helper T cells and cytotoxic T cells. The importance of regulatory T cells (T_{reg}) characterized by the expression of CD4⁺, CD25⁺, and Foxp3⁺, has been established. T_{reg} cells are known to support immunological tolerance, also known as immune homeostasis, by reducing exaggerated immune responses and preserving resistance to autoantigens. However, when immune function is impaired due to an imbalance of T_{reg} cells and numerous autoantibodies are produced, they overwhelm anti-inflammatory mechanisms, leading to severe inflammation and tissue damage.

Symptoms may vary depending on the specific type of autoimmune disease in the patient. However, several types exhibit similar symptoms, one of which is fatigue. Other common symptoms include redness and swelling, and many patients report muscle pain. Additionally, hair loss is a side effect of some autoimmune diseases. Fever and numbness or tingling in the hands and feet are also common symptoms. In some situations, autoimmune diseases can lead to potentially harmful consequences.

Managing autoimmune diseases is much more difficult. Unlike cancer or infections, where the treatment goal is to eliminate harmful cells from the body, autoimmune diseases require maintaining the immune system. The goal is to reduce inflammation by rebuilding tissues and organs damaged by inflammation and resetting the immune system.

While there is no known cure for autoimmune diseases, symptoms can be managed. Therefore, traditional treatments for autoimmune diseases focus on reducing the signs and symptoms of the disease and limiting the autoimmune process. Some NSAIDs and TNF blockers can be used to treat autoimmune diseases, but they primarily help reduce pain in the joints, muscles, and bones. Immunosuppressants, such as corticosteroids, are often recommended to reduce the severity of damage caused by abnormal immune system function. However, immunosuppressants, currently considered as gold standard for treating autoimmune diseases, are mostly associated with harmful side effects, and long-term use of these drugs may increase the risk of fatal infections and cancer.

5-Benzylaminosalicylic acid compounds or pharmaceutically acceptable salts thereof have been used in the treatment of neurodegenerative diseases (US Patent No. 6,964,982). In previous studies, 2-hydroxy-5-[2(4-trifluoromethyl-phenyl)ethylamino]benzoic acid is a potent spin-trapping molecule and microsomal prostaglandin E synthase-1 (mPGES-1) inhibitor at nanomolar concentrations, and has been shown to block neuronal death, lateral sclerosis, and autophagocytosis formation, as well as activate motor function and increase lifespan in a mouse model of amyotrophic lateral sclerosis.

### DISCLOSURE

### Technical Problem

5-Benzylaminosalicylic acid compounds or pharmaceutically acceptable salts thereof have been derived for the treatment of autoimmune diseases in various animal models of autoimmune diseases. Surprisingly, the administration of 2-hydroxy-5-[2-(4-trifluoromethylphenyl)ethylamino]benzoic acid showed significantly beneficial effects in various animal models of autoimmune diseases. In models of systemic lupus erythematosus (SLE) and multiple sclerosis (MS) associated with autoimmune diseases effects such as improved behavioral changes, protective effects against cell damage in the spleen and liver, restoration of abnormal immune responses, and reduction of inflammatory responses were observed. In animal models of psoriasis, effects such as inhibition of the increase in the severity of skin lesions, reduction in epidermal thickness, compression of collagen patterns, and reduction in spleen index were observed. Therefore, 2-hydroxy-5-[2-(4-trifluoromethylphenyl)ethylamino]benzoic acid can be a potential therapeutic choice for treating autoimmune diseases by inhibiting both oxidative stress and inflammation.

If useful therapeutic substances capable of treating or preventing autoimmune diseases are developed, they will be of great help in treating patients by reducing pain and inflammatory responses, regulating the immune system, and inhibiting apoptosis without side effects.

### Technical Solution

The present invention provides the use of a composition comprising 5-benzylamino salicylic acid compound represented by Chemical Formula (I) or a pharmaceutically acceptable salt thereof, for treating and/or preventing autoimmune diseases such as systemic lupus erythematosus (SLE), multiple sclerosis (MS), celiac disease, Type 1 diabetes mellitus, Graves' disease, inflammatory bowel disease, alopecia areata, Addison's disease, local or systemic scleroderma, psoriasis, atopic dermatitis, dermatomyositis, vitiligo, primary biliary cirrhosis, psoriatic arthritis, ankylosing spondylitis, reactive arthritis or Reiter's syndrome, rheumatoid arthritis, chronic thyroiditis (Hashimoto's thyroiditis or autoimmune thyroiditis), autoimmune hemolytic anemia, immune thrombocytopenia, Sjogren's syndrome, autoimmune retinopathy, acute anterior uveitis, myasthenia gravis, autoimmune vasculitis, antiphospholipid antibody syndrome, SAPHO syndrome, adult-onset Still's disease, and Behcet's disease.

The present invention also provides a use of a composition comprising 5-benzylamino salicylic acid compound represented by Chemical Formula (I) or a pharmaceutically acceptable salt thereof, for the manufacture of a medicine for treating and/or preventing the above-mentioned autoimmune diseases.

### Advantageous Effects

Systemic lupus erythematosus (SLE) and multiple sclerosis (MS) are representative autoimmune diseases. Using an experimental autoimmune encephalomyelitis (EAE) model, one of the animal models of SLE and MS, the effects of 5-benzylamino salicylic acid compounds of Chemical Formula (I) (specifically, compound 2: 2-hydroxy-5-[2-(4-trifluoromethyl-phenyl)ethylamino]benzoic acid) were confirmed. The results showed that the size of the spleen and splenic follicles was significantly reduced, and in the SLE model, the anti-dsDNA titer, a key biomarker of SLE, was significantly reduced; CD206 (an anti-inflammatory phase marker of M2 macrophages) expression was increased; and the expressions of iNOS (inducible Nitric Oxide Synthase), Tumor Necrosis Factor (TNF)-α, and IFN (Interferon)-γ (an inflammation stage marker) were reduced, and the T_{reg} cell population was significantly increased, confirming that compound 2 exhibits therapeutic effects through immunomodulatory effects (maintaining balance of T_{reg} cells) as well as anti-inflammatory and antioxidant effects. Therefore, 5-benzylamino salicylic acid compounds of Chemical Formula (I) (specifically, compound 2: 2-hydroxy-5-[2-(4-trifluoromethylphenyl)ethylamino]benzoic acid) can be usefully employed for autoimmune diseases.

In addition, psoriasis is one of the representative autoimmune diseases. Using an imiquimod (IMQ)-induced psoriasis mouse model, which is known as a psoriasis disease model, the effects of 5-benzylamino salicylic acid compounds of Chemical Formula (I) (specifically, compound 2: 2-hydroxy-5-[2-(4-trifluoromethyl-phenyl)ethylamino]benzoic acid) were confirmed. As a result, it was confirmed that it exhibits therapeutic effects on psoriasis, such as inhibiting the increase in the severity of skin lesions, reducing the thickness of hypertrophied epidermis, compressing collagen patterns, and reducing the spleen index. Therefore, 5-benzylamino salicylic acid compounds of Chemical Formula (I) (specifically, compound 2: 2-hydroxy-5-[2-(4-trifluoromethyl-phenyl)ethylamino]benzoic acid) can be usefully employed in the treatment of autoimmune diseases, particularly psoriasis.

### DESCRIPTION OF DRAWINGS

**Figures 1a** and **1b** show the spleen size and spleen index of systemic lupus erythematosus (SLE) mice.
**Figures 2a** to **2c** show the histological evaluation of the spleen in SLE mice.
**Figure 3** shows the results of measuring anti-double-stranded DNA (anti-dsDNA) antibodies in SLE mouse serum.
**Figure 4** shows the clinical scores associated with an experimental autoimmune encephalomyelitis (EAE) mouse model.
**Figure 5** shows the size of the spleen in an EAE mouse.
**Figure 6** shows the expression of CD206, iNOS, tumor necrosis factor (TNF)-α, and IFN-γ in the spleen of EAE mice.
**Figure 7** shows the ratio of regulatory T cells (T_{reg}) in the spleen of EAE mice.
**Figures 8a** and **8b** to **8e** show keratinized lesions and the Psoriasis Area and Severity Index (PASI) of psoriasis mice.
**Figures 9a** and **9b** show the histological evaluation (H&E staining) and epidermal thickness in psoriasis mice.
**Figure 10** shows the histological evaluation (Masson's trichrome staining) in psoriasis mice.
**Figures 11a** and **11b** show spleen size and spleen index in psoriatic mice.

### BEST MODE

The present invention provides a pharmaceutical composition and method for the prevention, reduction of the risk of onset or treatment of autoimmune diseases such as systemic lupus erythematosus (SLE), multiple sclerosis (MS), celiac disease, Type 1 diabetes mellitus, Graves' disease, inflammatory bowel disease, alopecia areata, Addison's disease, local or systemic scleroderma, psoriasis, atopic dermatitis, dermatomyositis, vitiligo, primary biliary cirrhosis, psoriatic arthritis, ankylosing spondylitis, reactive arthritis or Reiter's syndrome, rheumatoid arthritis, chronic thyroiditis (Hashimoto's thyroiditis or autoimmune thyroiditis), autoimmune hemolytic anemia, immune thrombocytopenia, Sjogren's syndrome, autoimmune retinopathy, acute anterior uveitis, myasthenia gravis, autoimmune vasculitis, antiphospholipid antibody syndrome, SAPHO syndrome, adult-onset Still's disease, and Behcet's disease, the pharmaceutical composition comprising: a 5-benzylaminosalicylic acid compound represented by Chemical Formula (I) or a pharmaceutically acceptable salt thereof: 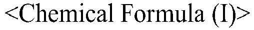 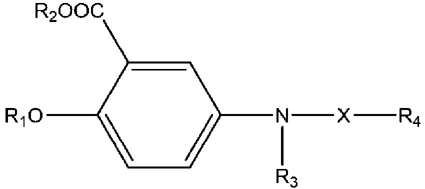 wherein,
X is selected from CO, SO₂ and (CH₂)ₙ;
R₁ is selected from hydrogen, C₁-C₆ alkyl and C₁-C₆ alkanoyl;
R₂ is hydrogen or C₁-C₆ alkyl;
R₃ is hydrogen or C₁-C₅ alkanoyl group; and
R₄ is selected from a phenyl group , a phenoxy group , and a 5- to 10-membered aryl group optionally substituted with one or more independently selected from nitro, halogen, C₁-C₆ alkyl, C₁-C₆ haloalkyl, C₁-C₅ alkoxy and C₁-C₅ haloalkoxy;
n is an integer from 1 to 5;
or a pharmaceutically acceptable salt thereof.

This disclosure provides a method useful for the treatment and/or prevention of autoimmune diseases such as systemic lupus erythematosus (SLE), multiple sclerosis (MS), celiac disease, Type 1 diabetes mellitus, Graves' disease, inflammatory bowel disease, alopecia areata, Addison's disease, local or systemic scleroderma, psoriasis, atopic dermatitis, dermatomyositis, vitiligo, primary biliary cirrhosis, psoriatic arthritis, ankylosing spondylitis, reactive arthritis or Reiter's syndrome, rheumatoid arthritis, chronic thyroiditis (Hashimoto's thyroiditis or autoimmune thyroiditis), autoimmune hemolytic anemia, immune thrombocytopenia, Sjogren's syndrome, autoimmune retinopathy, acute anterior uveitis, myasthenia gravis, autoimmune vasculitis, antiphospholipid antibody syndrome, SAPHO syndrome, adult-onset Still's disease, and Behcet's disease, comprising administering the compounds of Chemical Formula (I) to a patient in need thereof.

In one embodiment, a number of compounds of Chemical Formula (I) were prepared and evaluated. In one embodiment, the composition and method comprise 5-benzylaminosalicylic acid compounds of Chemical Formula (I) or a pharmaceutically acceptable salt thereof.

In one embodiment, 5-benzylaminosalicylic acid compound is 5-benzylaminosalicylic acid itself.

Preferred examples of 5-benzylaminosalicylic acid compounds include 2-hydroxy-5-phenethylamino-benzoic acid (compound 1), 2-hydroxy-5-[2-(4-trifluoromethyl-phenyl)-ethylamino]-benzoic acid (compound 2), 2-hydroxy-5-[2-(3-trifluoromethyl-phenyl)-ethylamino]-benzoic acid (compound 3), 5-[2-(3,5-bis-trifluoromethyl-phenyl)-ethylamino]-2-hydroxy-benzoic acid (compound 4), 2-hydroxy-5-[2-(2-nitro-phenyl)-ethylamino]-benzoic acid (compound 5), 5-[2-(4-chloro-phenyl)-ethylamino]-2-hydroxy-benzoic acid (compound 6), 5-[2-(3,4-difluoro-phenyl)-ethylamino]-2-hydroxy-benzoic acid (compound 7), 5-[2-(3,4-dichloro-phenyl)-ethylamino]-2-hydroxy-benzoic acid (compound 8), 5-[2-(4-fluoro-2-trifluoromethyl-phenyl)-ethylamino]-2-hydroxy-benzoic acid (compound 9), 5-[2-(2-fluoro-4-trifluoromethyl-phenyl)-ethylamino]-2-hydroxy-benzoic acid (compound 10), 2-hydroxy-5-[2-(4-methoxy-phenyl)-ethylamino]-benzoic acid (compound 11), 2-hydroxy-5-(2-o-tolyl-ethylamino)-benzoic acid (compound 12), 2-hydroxy-5-(3-phenyl-propylamino)-benzoic acid (compound 13), 2-hydroxy-5-[3-(4-trifluoromethyl-phenyl)-propylamino]-benzoic acid (compound 14), 5-[3-(4-fluoro-phenyl)-propylamino]-2-hydroxy-benzoic acid (compound 15), 5-[3-(3,4-dichloro-phenyl)-propylamino]-2-hydroxy-benzoic acid (compound 16), 2-hydroxy-5-(3-p-tolyl-propylamino)-benzoic acid (compound l7), 2-acetoxy-5-[2-(4-trifluoromethyl-phenyl)-ethylamino]-benzoic acid (compound 18), 5-[2-(2-chloro-phenyl)-ethylamino]-2-hydroxy-benzoic acid (compound 19), 5-benzylaminosalicylic acid (compound 20), 5-(4-nitrobenzyl)aminosalicylic acid (compound 21), 5-(4-chlorobenzyl)aminosalicylic acid (compound 22), 5-(4-trifluoromethylbenzyl)aminosalicylic acid (compound 23), 5-(4-fluorobenzyl)aminosalicylic acid (compound 24), 5-(4-methoxybenzyl)aminosalicylic acid (compound 25), 5-(2,3,4,5,6-pentafluorobenzyl)aminosalicylic acid (compound 26), 5-(4-nitrobenzyl)amino-2-hydroxy ethylbenzoate (compound 27), 5-(4-nitrobenzyl)-N-acetylamino-2-hydroxy ethylbenzoate (compound 28), 5-(4-nitrobenzyl)-N-acetylamino-2-acetoxy ethylbenzoate (compound 29), 5-(4-nitrobenzoyl)aminosalicylic acid (compound 30), 5-(4-nitrobenzenesulfonyl)aminosalicylic acid (compound 31), 5-[2-(4-nitrophenyl)-ethyl]aminosalicylic acid (compound 32), and 5-[3-(4-nitro-phenyl)-n-propyl]aminosalicylic acid (compound 33), but not limited thereto. In a specific preferred embodiment, the compound of Chemical Formula (I) is compound 2, 2-hydroxy-5-[2-(4-trifluoromethylphenyl)ethylamino]benzoic acid or a pharmaceutically acceptable salt thereof.

The 5-benzylaminosalicylic acid compound or a pharmaceutically acceptable salt thereof of the present disclosure may be prepared by the reaction scheme described in U.S. Patent No. 6,573,402, but not limited thereto.

In one embodiment, altered inflammatory response, abnormal immune response, cell damage of multiple organs, and behavioral dysfunction such as tail paralysis and waddling gait are symptoms of an autoimmune disease. In one embodiment, the compounds of Chemical Formula (I) reduce and/or inhibit abnormal immune response, cell damage of multiple organs, and behavioral dysfunction.

In one embodiment, thickened erythema and keratin lesions of the skin, epidermal hypertrophy (acanthosis) and increased thickness, abnormal keratinocyte differentiation, elongated rate ridges of the epidermis, infiltration of immune cells into the epidermis and dermis, irregular, fragmentary, and disordered collagen fiber structure, and an increase in spleen size and weight are symptoms of an autoimmune disease. In one embodiment, the compound of Chemical Formula (I) reduces and/or inhibits these abnormal immune responses, increased severity of skin lesions, increased epidermal thickness, disordering of collagen patterns, and increased spleen index.

In one embodiment, treatment of an autoimmune disease is achieved through the simultaneous pharmacological inhibition of oxidative stress and inflammation. In one embodiment, treatment of an autoimmune disease is achieved through inhibition of oxidative stress and prostaglandin E₂ synthesis. In one embodiment, treatment of an autoimmune disease is achieved through inhibition of oxidative stress and microsomal prostaglandin E synthase-1.

In one embodiment, the animal exhibits pathophysiological changes. In one embodiment, the pathophysiological changes are selected from an increase in follicle diameter, glomerular proliferation, leukocyte exudation, karyorrhexis and fibrinoid necrosis, a decrease in spleen follicles, cellular crescent, hyaline deposition for active index; interstitial inflammation, glomerular sclerosis, fibrous crescent, tubular atrophy, and interstitial fibrosis. In one embodiment, the pathophysiological changes are selected from an increase in macrophage size; an increase in the number of macrophages; and cell loss.

In one embodiment, the human patients are selected from those consisting of systemic lupus erythematosus (SLE), multiple sclerosis (MS), celiac disease, Type 1 diabetes mellitus, Graves' disease, inflammatory bowel disease, alopecia areata, Addison's disease, local or systemic scleroderma, psoriasis, atopic dermatitis, dermatomyositis, vitiligo, primary biliary cirrhosis, psoriatic arthritis, ankylosing spondylitis, reactive arthritis or Reiter's syndrome, rheumatoid arthritis, chronic thyroiditis (Hashimoto's thyroiditis or autoimmune thyroiditis), autoimmune hemolytic anemia, immune thrombocytopenia, Sjogren's syndrome, autoimmune retinopathy, acute anterior uveitis, myasthenia gravis, autoimmune vasculitis, antiphospholipid antibody syndrome, SAPHO syndrome, adult-onset Still's disease, and Behcet's disease.

In one embodiment, the animal is selected from the group consisting of cattle, horses, sheep, cats, chinchillas, dogs, ferrets, gerbil, pigs, and hamsters.

### Definition

The definitions of the terms described below apply when the term is used alone or in combination with other terms.

The term "acetoxy" means a group represented by the general chemical formula hydrocarbyl C(O)O-, preferably alkyl C(O)O-.

The term "alkyl" groups (including the 'alkyl' of haloalkyl) or "alkane" are fully saturated straight-chain or branched-chain type non-aromatic hydrocarbon. Generally, straight-chain or branched-chain alkyl groups have 1 to about 20 carbon atoms, preferably 1 to about 10, unless otherwise defined. C₁-C₆ straight-chain or branched-chain alkyl groups are also called "lower alkyl groups". In one embodiment, alkyl is C₁-C₅ alkyl, more preferably C₁-C₃ alkyl. More specifically, the preferred alkyl group is methyl, ethyl, propyl, isopropyl, butyl, sec-butyl, and tert-butyl, but not limited thereto.

Additionally, the term "alkyl" (or "lower alkyl") used throughout the specification, examples, and claims is intended to include both "unsubstituted alkyl" and "substituted alkyl", the latter referring to an alkyl residue having a substituent that replaces a hydrogen at one or more carbons of a hydrocarbon backbone. Unless otherwise specified, these substituents may comprise halogens, hydroxyl groups, carbonyl groups (e.g. carboxyl, alkoxycarbonyl, formyl, or acyl such as alkyl C(O)), thiocarbonyl groups (e.g. thioester, thioacetate, or thioformate), alkoxy, phosphoryl, phosphate, phosphonate, phosphinate, amino, amido, amidin, imine, cyano, nitro, azido, silyl ether, sulfhydryl, alkylthio, sulfate, sulfonate, sulfamoyl, sulfonamido, sulfonyl, heterocyclyl, aralkyl, or aromatic or heteroaromatic residues. It will be understood by those skilled in the art that a residue substituted on the hydrocarbon chain may substitute itself where appropriate. For example, the substituent of a substituted alkyl may include substituted and unsubstituted forms such as amino, azido, imino, amido, phosphoryl (including phosphonates and phosphinates), sulfonyl (including sulfates, sulfonamido, sulfamoyl, and sulfonates), and silyl groups, as well as ethers, alkylthiols, carbonyls (including ketones, aldehydes, carboxylates, and esters), -CF₃, -CN, etc. Exemplary examples of substituted alkyls are described below. Cycloalkyls can be further substituted with alkyl, alkenyl, alkoxy, alkylthio, aminoalkyl, carbonyl -substituted alkyl, -CF₃, -CN, etc.

The term "C_{x-y}" means that when used with chemical residues such as acyl, acyloxy, alkyl, alkenyl, alkynyl, or alkoxy, it includes a group in which carbons in the chain are included from x to y. For example, the term "C_{x-y}alkyl" refers to a substituted or unsubstituted saturated hydrocarbon group including a straight-chain alkyl group and a branched-chain alkyl group including carbons from x to y in the chain, including haloalkyl groups such as trifluoromethyl and 2,2,2-trifluoroethyl. C0 alkyl indicates hydrogen when the group is at a terminal position, and a bond when it is internal. The terms "C_{2-y}alkenyl" and "C_{2-y}alkynyl" refer to a substituted or unsubstituted unsaturated aliphatic group that is similar in length to the alkyl described above and is substitutable, but includes one or more double or triple bonds, respectively.

The term "alkanoyl" refers to a group represented as hydrocarbyl-C(O)-, preferably alkyl-C(O)-.

The term "alkoxy" (including "alkoxy" of haloalkoxy) means an oxygen-attached alkyl group, preferably a lower alkyl group. In one embodiment, preferably, the alkoxy is C₁-C₅ alkoxy, more preferably C₁-C₃ alkoxy. More specifically, preferable alkoxy includes methoxy, ethoxy and propaneoxy, but not limited thereto. Halogens include, but are not limited to, fluorides, chlorides, bromides, and iodides. Preferably, the alkanoyl is C₂-C₁₀ alkanoyl, more preferably C₃-C₅ alkanoyl. More specifically, a desirable alkanoyl includes, but not limited to, ethanoyl, propanoyl, and cyclohexanecarbonyl.

The terms "amine" and "amino" are recognized in the art and refer to unsubstituted and substituted amines and their salts, for example, parts that can be represented as follows.

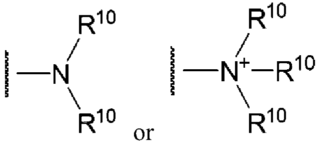

Here, each R¹⁰ independently represents a hydrogen or hydrocarbyl group, or two R¹⁰s together with the N atoms to which they are attached complete a heterocycle having 4 to 8 atoms within a ring structure.

The term "aryl" as used in this specification comprises a substituted or unsubstituted monocyclic aromatic group in which each atom of the ring is a carbon. Preferably the ring is a 5- to 10-membered ring, more preferably a 6- to 10-membered ring or a 6-membered ring. The term "aryl" also comprises a polycyclic ring system having two or more cyclic rings in which two or more carbons are common to two adjacent rings, wherein at least one of the rings is aromatic, and, for example, the other cyclic ring may be a cycloalkyl, cycloalkenyl, cycloalkynyl, aryl, heteroaryl, and/or heterocyclyl. Aryl groups include benzene, naphthalene, phenanthrene, phenol, aniline, etc. Exemplary substitutions of the aryl group may include, for example, halogens, haloalkyls such as trifluoromethyl , hydroxyl, carbonyls (e.g. carboxyl, alkoxycarbonyl, formyl, or acyls such as alkylC(O)), thiocarbonyls (e.g. thioesters, thioacetates, or thioformates), alkoxyl, phosphoryl, phosphate, phosphonate, phosphinate, amino, amido, amidin, imine, cyano, nitro, azido, silyl ether, sulfhydryl, alkylthio, sulfate, sulfonate, sulfamoyl, sulfonamido, sulfonyl, heterocyclyl, aralkyl, or aromatic or heteroaromatic residues.

The terms "halo" and "halogen" as used in this specification mean halogen and include chloro, fluoro, bromo, and iodo.

The term "lower" is used with chemical residues such as acyl, acyloxy, alkyl, alkenyl, alkynyl, or alkoxy, it means comprising a group having 10 or fewer, preferably 6 or fewer, non-hydrogen atoms in the substituent. For example, "lower alkyl" refers to an alkyl group including 10 or fewer, preferably 6 or fewer, carbon atoms. In certain embodiments, the acyl, acyloxy, alkyl, alkenyl, alkynyl, or alkoxy substituents defined herein are each a lower acyl, lower acyloxy, lower alkyl, lower alkenyl, lower alkynyl, or lower alkoxy, whether appearing alone or in combination with other substituents. For example, as in the mention of hydroxyalkyl and aralkyl (in this case, for example, atoms within the aryl group are not counted when calculating carbon atoms of alkyl substituents).

The term "substituted" refers to a portion having a substituent that replaces a hydrogen at one or more carbons of the skeleton. "Substitution" or "substituted" is to be understood to include the implicit condition that such substitution is subject to the allowable valence of the substituted atom and the substituent, and that the substitution results in a stable compound that is not spontaneous to undergo modifications such as rearrangement, cyclization, or elimination. The term "substituted" as used in this specification is considered to include all allowable substituents of an organic compound. In a broad sense, allowable substituents include acyclic and cyclic, branched and unbranched, carbon-cyclic and heterocyclic, aromatic and non-aromatic types of organic compounds. Allowable substituents are one or more for a suitable organic compound and may be the same or different. For the purposes of the present invention, a heteroatom such as nitrogen may have a hydrogen substituent and/or any allowable substituent of the organic compound described herein that satisfies the valence of the heteroatom. The substituents may include any of the substituents described herein, e.g., halogen, haloalkyl, hydroxyl, carbonyl (e.g., carboxyl, alkoxycarbonyl, formyl, or acyl), thiocarbonyl (e.g., thioester, thioacetate, or thioformate), alkoxy, phosphoryl , phosphate, phosphonate, phosphinate, amino, amido, amidin, imine, cyano, nitro, azido, sulfhydryl, alkylthio, sulfate, sulfonate, sulfamoyl, sulfonamido, sulfonyl, heterocyclyl, aralkyl, or aromatic or heteroaromatic residues. It will be understood by those skilled in the art that, where appropriate, the substituents themselves may be substituted. Unless specifically stated as "unsubstituted", references to chemical residues herein are understood to include substituted variations. For example, references to an "aryl" group or residue implicitly include both substituted and unsubstituted variations.

In the present invention, "pharmaceutically acceptable salt" means a salt produced by an acid or base that is non-toxic or has little to no toxicity. If the compound of the present disclosure is acidic, a base addition salt of the compound of the present disclosure may be prepared by reacting the free base of the compound with a sufficient amount of a preferred base and a suitable inert solvent. Pharmaceutically acceptable base addition salts include, but are not limited to, salts made of sodium, potassium, calcium, ammonium, magnesium, or organic aminos. If the compound of the present invention is basic, an acid addition salt of the compound may be prepared by reacting the free base of the compound with a sufficient amount of a preferred acid and a suitable inert solvent. Pharmaceutically acceptable acid addition salts include, but are not limited to, propionic acid, isobutyric acid, oxalic acid, malic acid, malonic acid, benzoic acid, succinic acid, suberic acid, fumaric acid, mandelic acid, phthalic acid, benzenesulfonic acid, p-tolylsulfonic acid, citric acid, tartaric acid, methanesulfonic acid, hydrochloric acid, hydrobromic acid, nitric acid, carbonic acid, monohydrogenated carbonic acid, phosphoric acid, monohydrogenated phosphoric acid, dihydrogenated phosphoric acid, sulfuric acid, monohydrogenated sulfuric acid, hydrogen iodide, and phosphoric acid. Additionally, pharmaceutically acceptable salts of the present invention include, but are not limited to, amino acid salts such as alginates, and organic acid analogs such as glucuronic acid or galactunoric acid.

For example, a pharmaceutically acceptable salt of 2-hydroxy-5-[2-(4-trifluoromethyl-phenyl)-ethylamino]-benzoic acid (compound 2), which is a preferred example of the present invention, can be prepared by the following reaction scheme. However, the following reaction method is provided by way of example and is not intended to limit the scope of the present disclosure.

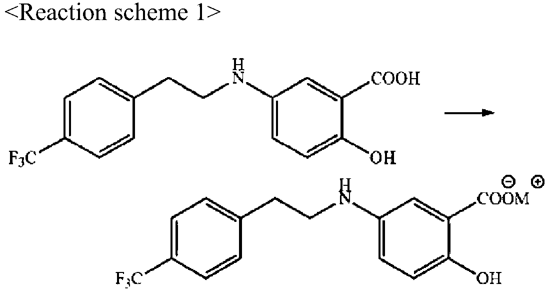

In the above reaction scheme, M is a pharmaceutically acceptable metal or basic organic compound such as diethylamine, lithium, sodium, and potassium.

More specifically, diethylamine salts can be prepared by dissolving a compound in alcohol, adding diethylamine dropwise, stirring, distilling under vacuum, and adding ether to crystallize. Alkali metal salts can be prepared by adding inorganic reagents such as lithium hydroxide, sodium hydroxide, or potassium hydroxide to a solvent such as alcohol, acetone, or acetonitrile to produce the desired salt, followed by freeze-drying. Additionally, according to a similar method, lithium salts can be made from lithium acetate, sodium salts from 2-ethylhexanate or sodium acetate, and potassium salts from potassium acetate.

Some of the compounds of the present disclosure may be in a hydrated form, or may exist in a solvated or unsolved form. Some of the compounds according to the present disclosure may exist in a crystalline or amorphous form, and any physical form is included within the scope of the present disclosure. Additionally, some of the compounds of the present disclosure may include one or more asymmetric carbon atoms or double bonds and exist in two or more stereoisomer forms, such as racemics, enantiomers, diastereomers, geometric isomers, etc. The present disclosure includes their individual stereoisomers of the compounds.

The present invention also provides a food composition for improving symptoms of an autoimmune disease, comprising a compound of Chemical Formula (I) or a pharmaceutically acceptable salt thereof.

The present disclosure is described in more detail below to help those skilled in the art understand it. However, the following examples are illustrative and are not intended to limit the scope of the present disclosure. It is evident that various modifications can be made without departing from the spirit and scope of the present disclosure or without sacrificing all material advantages of the present disclosure.

### Example 1. Evaluation of the symptom-improving effect of compound 2 in an SLE mouse model

Compound 2, 2-hydroxy-5-[2-(4-trifluoromethyl-phenyl)ethylamino]benzoic acid was used as a representative compound.

The goal of this Example was to investigate the effects of compound 2 in the imiquimod (IMQ)-induced lupus model, which is widely used as one of the induced SLE models.

### Experimental design

Female BALB/C mice weighing 18-20 g were supplied by Saeron Bio Co., Ltd. (Korea) and acclimatized for 7 days. The animals were housed in separate cages with free access to laboratory feed and water. During the experiment, the animals were maintained at 23±3°C and 50±10% relative humidity with a 12-hour light-dark cycle. The animals were treated according to a protocol approved by the Animal Ethics Committee of GNT Pharmaceuticals. The mice were randomly divided into four groups: a normal group (n=6), a control SLE group (vehicle group, n=8), an IMQ+Compound 2 group (Compound 2 treatment group, n=4), and an IMQ+N-acetylcysteine group (NAC treatment group, n=8). All IMQ treatment groups were administered 5% imiquimod cream (Aldara Cream, Dong-A Pharmaceutical Co., Ltd.) topically to the right ear at a dose of 1.25 mg daily every other day for 4 weeks. Compound 2 (5 mg/kg, twice daily) was orally administered to the IMQ+Compound 2 group for 4 weeks, followed by concurrent IMQ treatment. N-acetyl Cysteine (150 mg/kg, once daily, adjusted to pH 7.5-8.0) was administered intraperitoneally to the IMQ+NAC group for 4 weeks along with IMQ treatment.

Mice were anesthetized with pentobarbital at the end of the experiment. Blood was collected through cardiac blood sampling. Within 30 minutes of collection, serum was separated from whole blood using EDTA by centrifugation at 12,000 g for 10 minutes. The extracted spleen was weighed, then the tissue was heated to 60°C, washed with xylene, and immersed in ethanol at gradient concentrations. The sections were H&E stained (BBC Biochemical, Washington State Mount Vernon) and then imaging was performed under a microscope. Observation was conducted through five fields of view at 10x magnification, and the number of spleen alba pulps (follicles) was counted. In addition, the diameter of the spleen alba pulp was observed using a micrometer. The diameter of the alba pulp was measured vertically and horizontally, and the average was calculated. Using a commercially available enzyme-linked immunosorbent assay (ELISA) kit (LBIS Mouse anti-dsDNA ELISA, Fujifilm Wako Shibayagi Co., Ltd., Richmond, Virginia, USA) serum anti-dsDNA (IgG specific) concentration was analyzed.

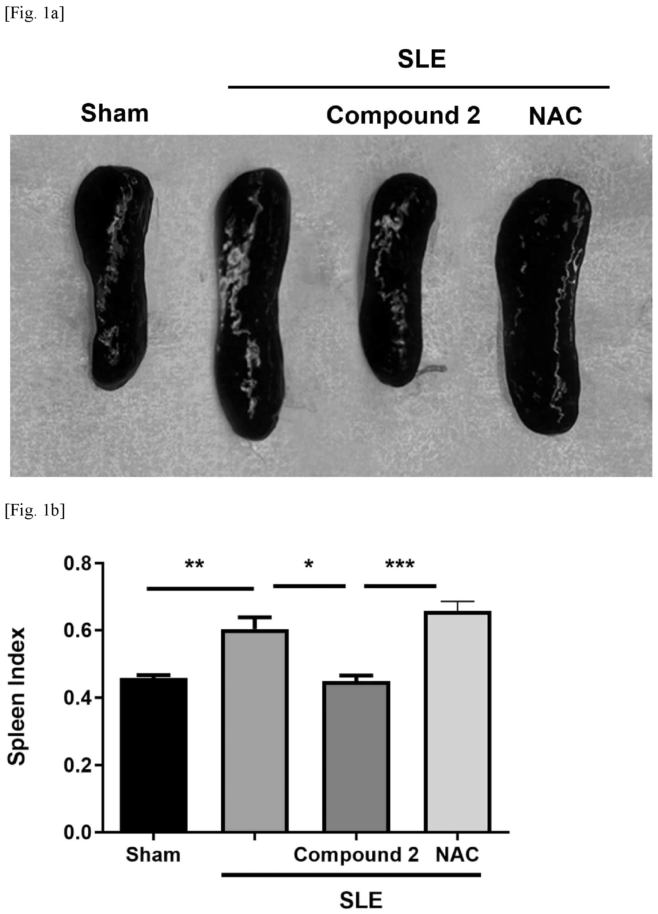

The spleen size of the control SLE group increased compared to the normal (Sham) group, whereas the spleen size of the compound 2-treated group was smaller than that of the control SLE group. However, the spleen size of the NAC-treated group was similar to that of the control SLE group (Fig. 1a). Regarding the spleen index (organ weight/body weight x 100%), the spleen index of the control SLE group was significantly higher than that of the normal group, while the spleen index of the compound 2-treated group was significantly lower than that of the control SLE group. However, there was no significant difference between the NAC-treated group and the control SLE group (Fig. 1b; ^{*}*P* <0.05, ^{**}*P* <0.01, ^{***}*P* <0.001).

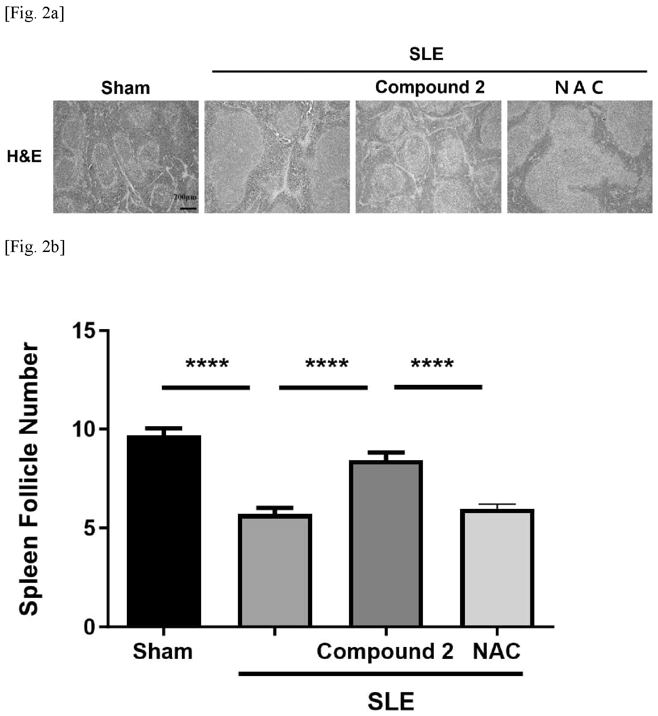

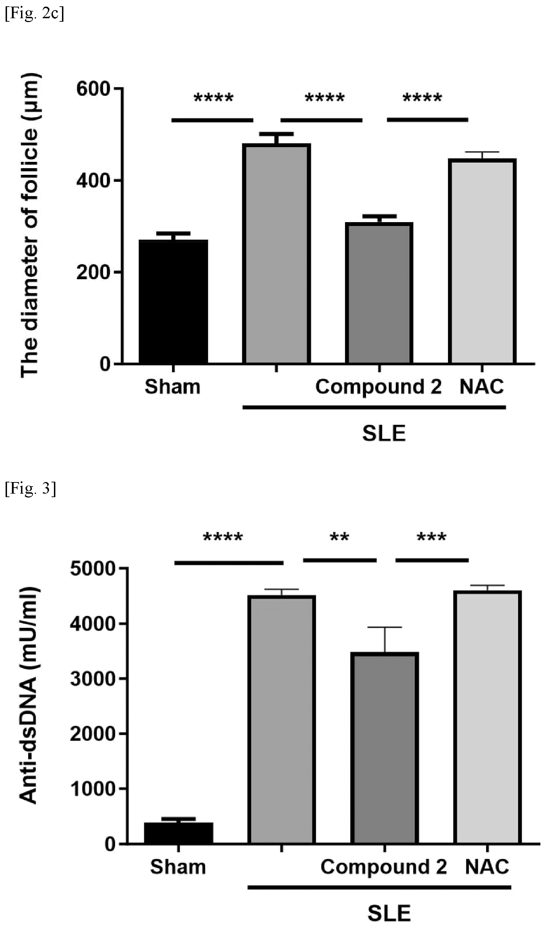

H&E stained cross-sections of the spleen in the control SLE group showed diluted and hypertrophied white pulp, indicating lymphatic hyperplasia due to autoimmune disease (Fig. 2a). In the group treated with compound 2 , the number of splenic follicles significantly increased and the follicle diameter significantly decreased, indicating that compound 2 has a cytoprotective effect against abnormal autoimmune-related splenic injury (Figs. 2b-2c, ^{****}*P* <0.0001). However, the group treated with N- acetylcysteine did not show a beneficial effect.

The anti-dsDNA concentration in the control SLE group was significantly increased compared to the normal group. In SLE mice, compound 2 significantly reduced anti-dsDNA titers, whereas NAC had no effect (Fig. 3; ^{**}*P* <0.01, ^{***}*P* <0.001 ^{****}*P* <0.0001).

### Example 2. Evaluation of the symptom-improving effect of compound 2 in an EAE mouse model

Multiple sclerosis (MS) is a neurological autoimmune disease in which the body's immune system attacks the cells of myelin, the protective sheath surrounding the ganglia of the brain and spinal cord. Until now, EAE has been well known as a preclinical model associated with multiple sclerosis. Therefore, an EAE mouse model was used to confirm the effects of compound 2 on autoimmune diseases.

### Experimental design

The EAE mouse model was induced in 6-week-old female C57BL/6 mice. Mice were divided into three groups: 6 mice were placed in the normal group, and 8 mice were placed in the control EAE group (vehicle group) and the EAE + Compound 2 group (Compound 2 treatment group), respectively. In the mice, 200 µg of oligodendrocyte glycoprotein peptide myelin oligodendrocyte glycoprotein 35-55 (MOG35-55, Prospecbio , Israel) emulsified in 400 µg of complete Freund's adjuvant (CFA, Sigma, USA) was inoculated. The emulsion was injected into both sides of each mouse. Subsequently, 200 ng of pertussis toxin (Sigma, USA) was injected intraperitoneally on days 0 and 2. Compound 2 was injected intraperitoneally at a dose of 3.3 mg/kg every 24 hours for 7 days starting on day 9. In the control EAE group, PBS was injected intraperitoneally. Clinical signs associated with EAE were monitored daily and rated on a scale of 0 to 5: Grade 0, no apparent clinical symptoms; Grade 0.5, partial tail paralysis; Grade 1, tail paralysis or waddling gait; Grade 1.5, partial tail paralysis and waddling gait; Grade 2, tail paralysis and waddling gait; Grade 2.5, partial quadriplegia; Grade 3, unilateral limb paralysis; Grade 3.5, unilateral paralysis and partial paralysis of the other limb; Grade 4, bilateral paralysis; Grade 4.5, coma; Grade 5, death.

On day 25 after EAE induction, mice were euthanized by exposure to CO₂, and spleens were extracted. RNA expression in the spleen was analyzed using Applied Biosystems^{™}QuantStudio 5 qPCR system (Thermofisher, USA) with AMPIGENE qPCR Green Mix Hyrox (Thermofisher, USA), including 400 nm forward and reverse primers (Bionics Korea) and SYBR green dye (Enzo Life Sciences, Farmingdale , NY, USA). The expression levels of each gene were standardized to glyceraldehyde 3-phosphate dehydrogenase (GAPDH) and compared to the expression levels of the normal or control EAE groups. To evaluate the effect of compound 2 on T_{reg}, mouse splenocyte preparations were stained with the T_{reg} detection kit (CD4/CD25/FoxP3) (Miltenyi Biotech, Germany) according to the manufacturer's instructions, and the T_{reg} population was evaluated using flow cytometry. The data was analyzed using FlowJo^{™} 10.8.1 software.

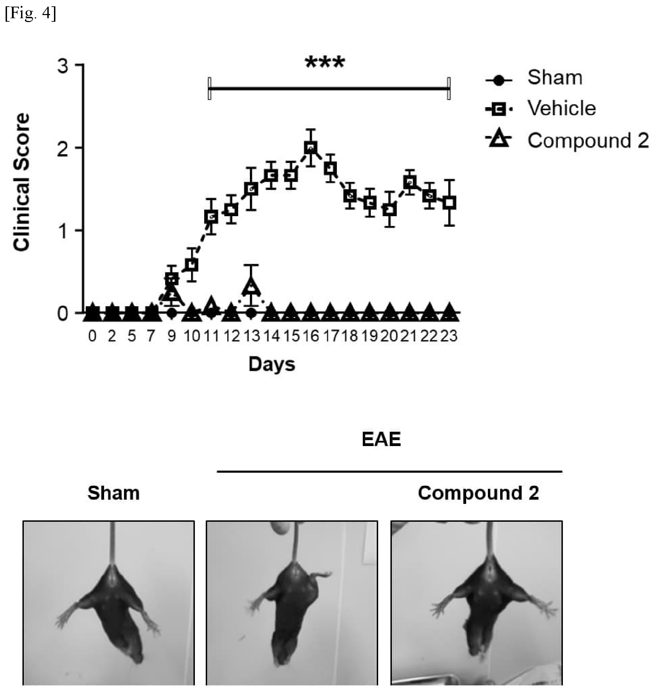

EAE-related neurological signs began on day 9, and symptom severity peaked on day 16. The onset date was similar between the control EAE group and the compound 2 treatment group. Clinical signs were effectively alleviated in the compound 2 treatment group (Fig. 4; ^{***}*P* <0.001).

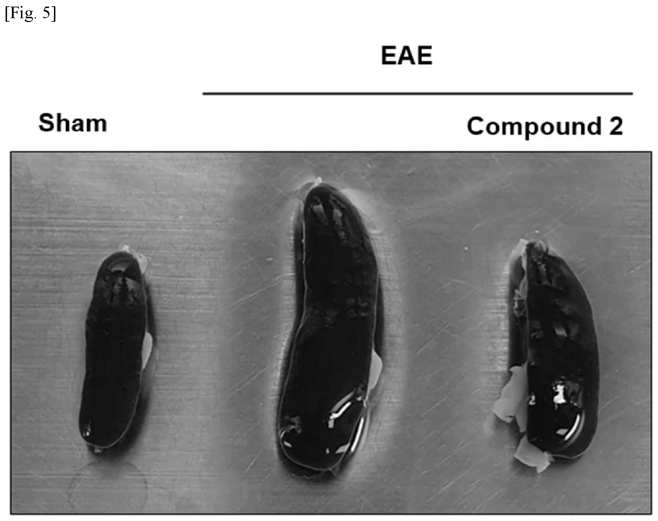

In the control EAE group, the size of the spleen increased, whereas the size of the spleen in the compound 2 treatment group was similar to that of the normal group (Fig. 5).

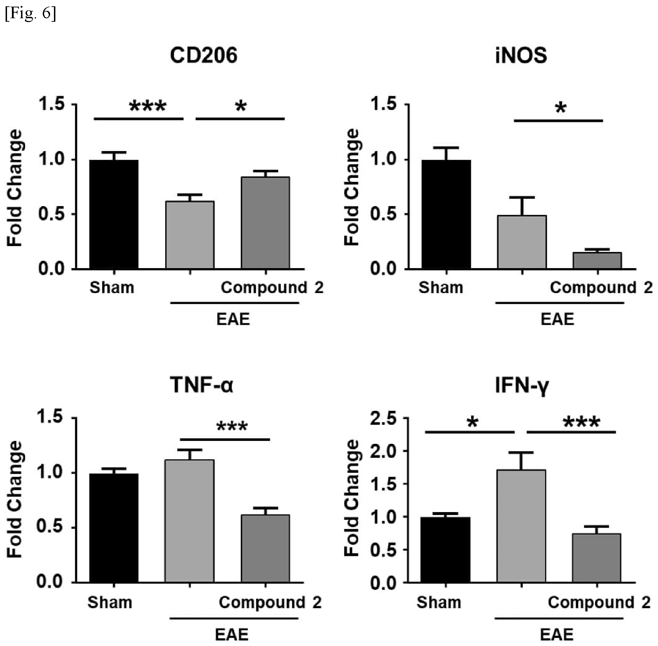

CD206 expression decreased in the control EAE group, but significantly increased in the compound 2-treated group compared to the control EAE group. iNOS expression was significantly lower in the compound 2-treated group compared to the control EAE group. The expression of inflammatory cytokines TNF-α and IFN-γ was lower in the compound 2 treatment group compared to the control EAE group (Fig. 6, ^{*}*P* <0.05, ^{***}*P* <0.001).

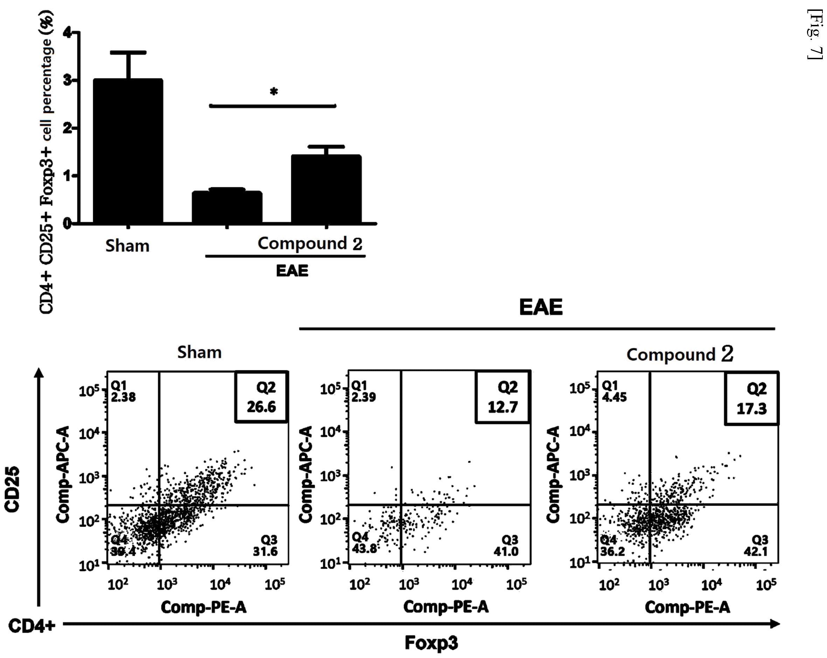

The proportion of CD4⁺CD25⁺Foxp3⁺ T_{reg} cells in the control EAE group was significantly lower compared to the normal group. However, the compound 2 treatment group had a significantly higher number of T_{reg} cells compared to the control EAE group (Fig. 7, ^{*}*P* <0.05).

### Conclusion of Example 1 and 2

To evaluate the therapeutic potential of compound 2, SLE and EAE, representative models of autoimmune diseases, were used in this experiment. The size of the spleen and splenic follicles was significantly alleviated in the compound 2-treated group. In the SLE model, the anti-dsDNA titer, a key biomarker of SLE, was significantly reduced in the compound 2-treated group. In the compound 2-treated group, the expression of CD206 (M2 anti-inflammatory phase marker) increased, while the expression of iNOS, TNF-α, and IFN-γ (inflammation phase markers) decreased. The T_{reg} cell population significantly increased in the compound 2-treated group. These results indicate that compound 2 possesses beneficial therapeutic effects through immunomodulatory effects (maintaining T_{reg} cell balance) as well as anti-inflammatory and antioxidant effects. Taken together, these findings strongly suggest that compound 2 can be applied to the treatment of autoimmune diseases.

### Example 3. Evaluation of the effect of compound 2 on improving skin lesions in a mouse psoriasis model

The goal of this Example was to investigate the effect of compound 2 in the imiquimod (IMQ)-induced psoriasis model, which is widely used as one of the induced psoriasis models.

### Model Description: Imiquimod (IMQ)-induced psoriasis model

For the experiment, psoriasis was induced using imiquimod (IMQ). The IMQ-induced psoriasis mouse model is one of the most widely used models for studying psoriasis. IMQ is a Toll-like receptor-7/8 (TLR-7/8) agonist used to treat actinic keratosis and basal cell carcinoma. It is known that topical application of IMQ cream to the skin of mice recruits immune cells and activates macrophages, monocytes, and dendritic cells to secrete inflammatory cytokines. The IMQ-induced psoriasis mouse model is known to be very similar to human psoriasis in terms of skin erythema, keratinization, epidermal changes (acanthosis), and inflammatory infiltration consisting of T cells, neutrophils, and dendritic cells.

### Experimental design

Male BALB/C mice weighing 20-22 g were supplied by Saeron Bio Co., Ltd. (Korea) and acclimatized for 7 days. The animals were housed in separate cages with free access to laboratory feed and water. During the experiment, the animals were maintained at 23±3°C and 50±10% relative humidity with a 12-hour light-dark cycle. The animals were treated according to a protocol approved by the Animal Ethics Committee of GNT Pharmaceuticals. The mice were randomly divided into three groups: a normal group (n=5), a control psoriasis group (Vehicle group, n=8), and an IMQ+Compound 2 group (compound 2 treatment group, n=9). All IMQ treatment groups were administered 5% imiquimod cream (Aldara Cream, Dong-A Pharmaceutical Co., Ltd.) topically to shaved backs at a dose of 62.5 mg per day for 6 days. Compound 2 (5 mg/kg, twice daily) was orally administered to the IMQ+Compound 2 group for 6 days, followed by IMQ treatment.

### Experimental results

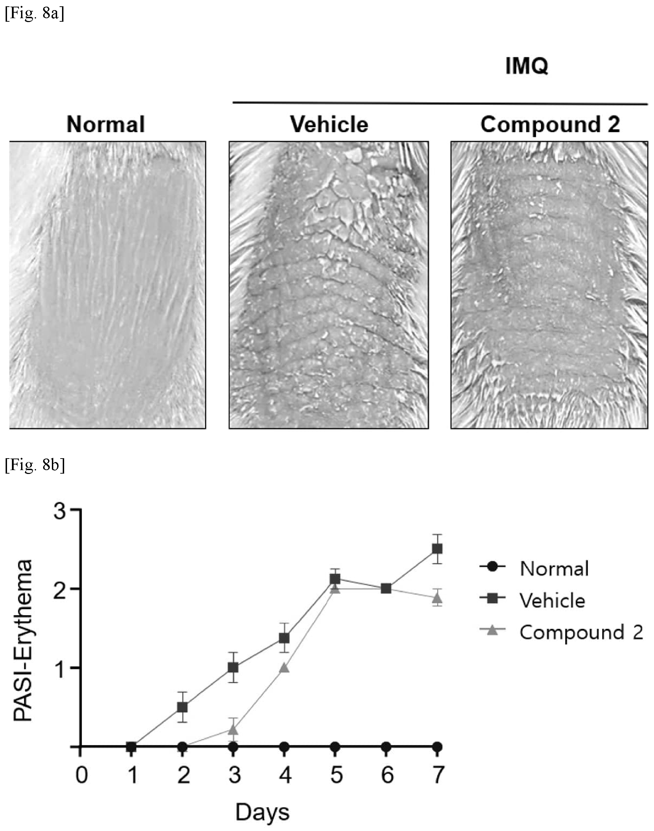

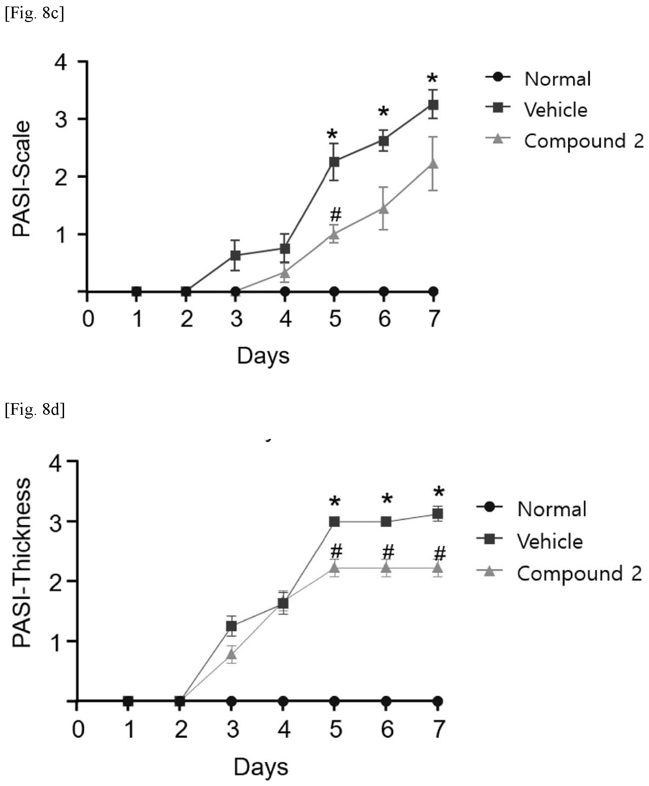

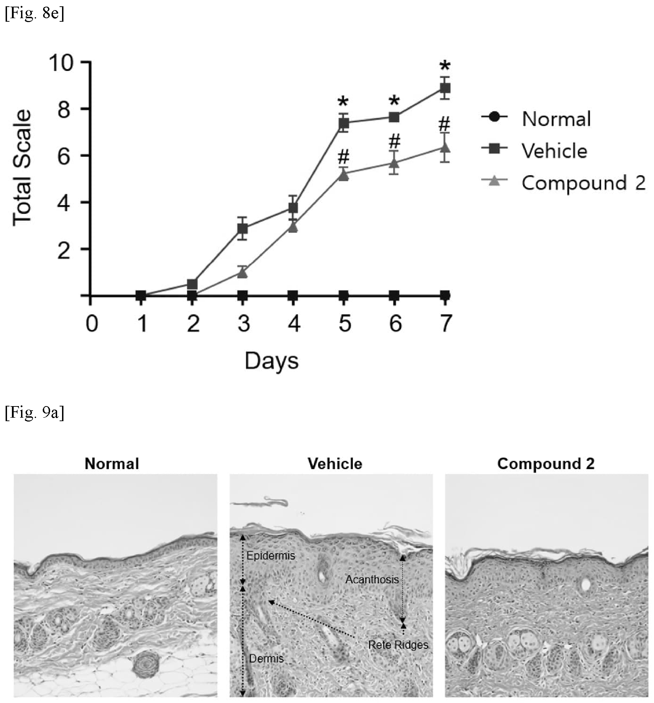

To evaluate the anti-psoriatic effects of compound 2, the severity of psoriasis was monitored using an IMQ-induced psoriasis mouse model. The Psoriasis Skin PASI score is used to assess the severity of skin lesions in mice. Mice treated with IMQ showed significantly thickened erythema and scales after 7 days compared to normal mice (Fig. 8a). In terms of PASI scores, the normal group was 0 points, whereas the control psoriasis group treated only with IMQ showed an increase in score (Figs. 8b~8e, ^{*}*P* <0.05 and control psoriasis group, ^{#}*P* <0.05 and compound 2 treatment group). However, the compound 2 treatment group showed a significantly lower score than the control psoriasis group. Treatment with compound 2 was shown to suppress the increase in severity of skin lesions caused by IMQ by reducing scales and thickness.

### Example 4. Histopathological changes following treatment with Compound 2 in IMQ-induced psoriasis mice

After confirming the therapeutic effect of compound 2 on psoriasis-like symptoms in mice, histological analysis was performed to identify histological changes occurring in psoriatic skin and methods of improvement. IMQ-induced inflammation exhibits histological characteristics of psoriasis, such as increased epidermal proliferation, abnormal keratinocyte differentiation, and elongated rate ridges of the epidermis.

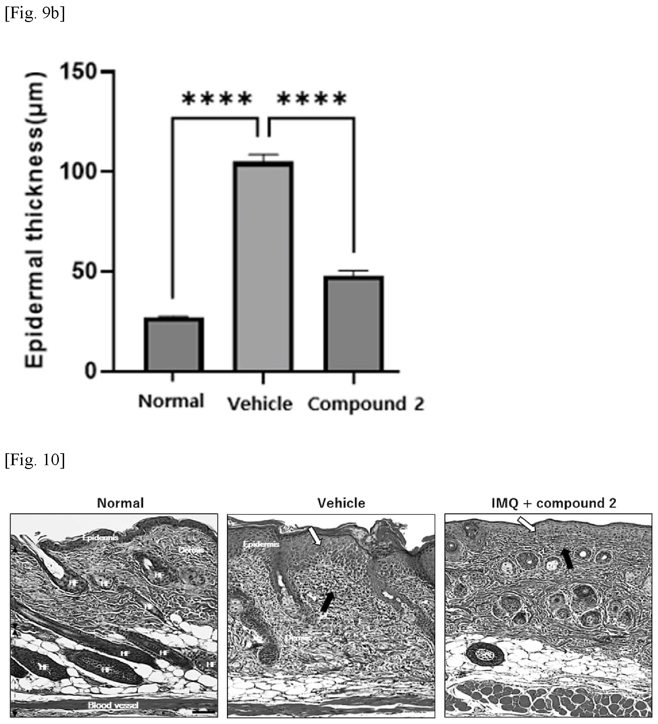

In the H&E-stained areas of the dorsal skin of the IMQ-induced control psoriasis group, epidermal hyperplasia, acanthosis, and elongated epidermal rate ridges were shown to be increased (Fig. 9a). In the control psoriasis group, epidermal thickness also increased compared to the normal group (Fig. 9b, ^{****}*P* <0.0001). In contrast, epidermal thickness was significantly reduced in the compound 2 treatment group. This increased epidermal thickness was due to the proliferation of basal cells and keratinocytes. In contrast to the IMQ alone treatment group (control psoriasis group), the histopathological manifestations of skin lesions in the compound 2 treatment group showed significant inhibition of epidermal thickening (acanthosis) and elongation of the rate ridges due to proliferation

The infiltration of immune cells into the epidermis and dermis is another pathological feature of psoriasis. Masson's trichrome (MT) staining showed that the application of IMQ significantly stimulated the proliferation of epidermal keratinocytes and induced the infiltration of inflammatory cells (Fig. 10). These features were not observed in the normal group. Treatment with compound 2 reduced IMQ-induced epidermal thickness and keratosis, and potently inhibited psoriasis-like inflammation. Furthermore, the normal group exhibited a long and regular reticular fiber structure, whereas the control psoriasis group showed an irregular, fragmentary, and disordered collagen fiber structure. However, mice treated with compound 2 displayed a much more compressed collagen pattern in the dermis (HF: Hair follicle, Open arrow: epidermal thickness, black arrow: inflammatory cell infiltration)

The above results indicate that compound 2 can alleviate symptoms that may occur in the skin of an imiquimod-induced psoriasis model and improve skin histopathological changes.

### Example 5. Evaluation of the effects of Compound 2 on splenic changes and splenic indices in IMQ-induced psoriasis mice

The spleen is an immune organ including various types of immune cells and plays an important role in maintaining the immune system. To confirm the systemic effects of IMQ inducing splenomegaly and the effects of compound 2 on the systemic immune response, the spleen index was evaluated.

Mice were anesthetized with pentobarbital at the end of the experiment. Blood was collected by cardiac blood sampling. Within 30 minutes of collection, serum was separated from whole blood using EDTA by centrifugation at 12,000 g for 10 minutes. The weight of the extracted spleen was measured.

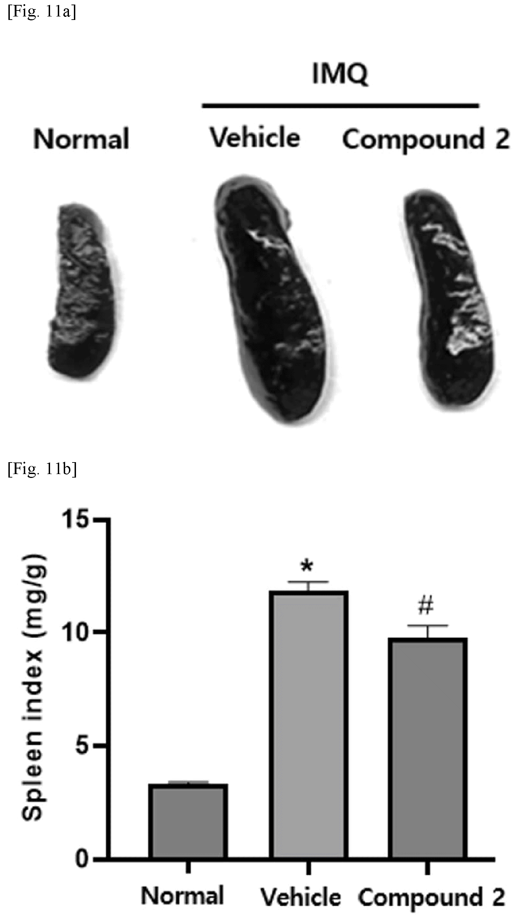

Application of IMQ increased spleen size and weight in IMQ-induced psoriasis mice (Fig. 11a). In contrast, treatment with compound 2 decreased spleen size and weight in IMQ-induced psoriasis mice. As a result, the spleen index decreased in the compound 2 group compared to the control psoriasis group, suggesting an anti-inflammatory effect of compound 2 (Fig. 11b, ^{*}*P* <0.05 and control psoriasis group, ^{#}*P* <0.05 and compound 2 treatment group).

The foregoing description of the present invention is for illustrative purposes only, and those skilled in the art will understand that other specific forms can be easily modified without altering the technical spirit or essential features of the present invention. Therefore, the Examples described above should be understood as illustrative in all respects and not restrictive. For example, each component described as a single unit may be implemented in a distributed manner, and components described as distributed may likewise be implemented in a combined form.

The scope of the present invention is defined by the claims set forth below, and all modifications or variations derived from the meaning and scope of the claims and equivalent concepts thereof should be interpreted as being included within the scope of the present invention.

## Claims

1. A pharmaceutical composition for preventing or treating an autoimmune disease, comprising a compound represented by the following Chemical Formula (I) or a pharmaceutically acceptable salt thereof: 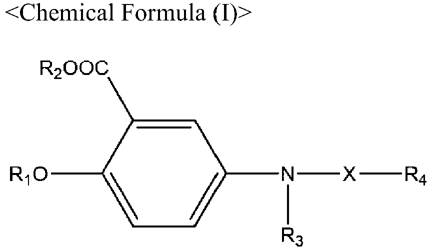 wherein,
X is selected from CO, SO₂ and (CH₂)ₙ;
R₁ is selected from hydrogen, C₁-C₆ alkyl and C₁-C₆ alkanoyl;
R₂ is hydrogen or C₁-C₆ alkyl;
R₃ is hydrogen or C₁-C₅ alkanoyl group; and
R₄ is selected from a phenyl group , a phenoxy group , and a 5- to 10-membered aryl group optionally substituted with one or more independently selected from nitro, halogen, C₁-C₆ alkyl, C₁-C₆ haloalkyl, C₁-C₅ alkoxy and C₁-C₅ haloalkoxy;
n is an integer from 1 to 5.

2. The pharmaceutical composition of Claim 1, wherein the compound of Chemical Formula (I) is selected from the group consisting of 2-hydroxy-5-phenethylamino-benzoic acid, 2-hydroxy-5-[2-(4-trifluoromethyl-phenyl)-ethylamino]-benzoic acid, 2-hydroxy-5-[2-(3-trifluoromethyl-phenyl)-ethylamino]-benzoic acid, 5-[2-(3,5-bis-trifluoromethyl-phenyl)-ethylamino]-2-hydroxy-benzoic acid, 2-hydroxy-5-[2-(2-nitro-phenyl)-ethylamino]-benzoic acid, 5-[2-(4-chloro-phenyl)-ethylamino]-2-hydroxy-benzoic acid, 5-[2-(3,4-difluoro-phenyl)-ethylamino]-2-hydroxy-benzoic acid, 5-[2-(3,4-dichloro-phenyl)-ethylamino]-2-hydroxy-benzoic acid, 5-[2-(4-fluoro-2-trifluoromethyl-phenyl)-ethylamino]-2-hydroxy-benzoic acid, 5-[2-(2-fluoro-4-trifluoromethyl-phenyl)-ethylamino]-2-hydroxy-benzoic acid, 2-hydroxy-5-[2-(4-methoxy-phenyl)-ethylamino]-benzoic acid, 2-hydroxy-5-(2-o-tolyl-ethylamino)-benzoic acid, 2-hydroxy-5-(3-phenyl-propylamino)-benzoic acid, 2-hydroxy-5-[3-(4-trifluoromethyl-phenyl)-propylamino]-benzoic acid, 5-[3-(4-fluoro-phenyl)-propylamino]-2-hydroxy-benzoic acid, 5-[3-(3,4-dichloro-phenyl)-propylamino]-2-hydroxy-benzoic acid, 2-hydroxy-5-(3-p-tolyl-propylamino)-benzoic acid, 2-acetoxy-5-[2-(4-trifluoromethyl-phenyl)-ethylamino]-benzoic acid, 5-[2-(2-chloro-phenyl)-ethylamino]-2-hydroxy-benzoic acid, 5-benzylaminosalicylic acid, 5-(4-nitrobenzyl)aminosalicylic acid, 5-(4-chlorobenzyl)aminosalicylic acid, 5-(4-trifluoromethylbenzyl)aminosalicylic acid, 5-(4-fluorobenzyl)aminosalicylic acid, 5-(4-methoxybenzyl)aminosalicylic acid, 5-(2,3,4,5,6-pentafluorobenzyl)aminosalicylic acid, 5-(4-nitrobenzyl)amino-2-hydroxy ethylbenzoate, 5-(4-nitrobenzyl)-N-acetylamino-2-hydroxy ethylbenzoate, 5-(4-nitrobenzyl)-N-acetylamino-2-acetoxy ethylbenzoate, 5-(4-nitrobenzoyl)aminosalicylic acid, 5-(4-nitrobenzenesulfonyl)aminosalicylic acid, 5-[2-(4-nitrophenyl)-ethyl]aminosalicylic acid, and 5-[3-(4-nitro-phenyl)-n-propyl]aminosalicylic acid.

3. The pharmaceutical composition of Claim 2, wherein the compound of Chemical Formula (I) is 2-hydroxy-5-[2-(4-trifluoromethyl-phenyl)ethylamino]benzoic acid or 2-acetoxy-5-[2-(4-trifluoromethyl-phenyl)ethylamino]benzoic acid.

4. The pharmaceutical composition of Claim 1, wherein the pharmaceutical composition comprises a capsule, a tablet including an orally disintegrating tablet, an orally disintegrating film, and an injectable including intraperitoneal and intravenous administration.

5. The pharmaceutical composition of Claim 1, wherein the autoimmune disease is selected from the group consisting of systemic lupus erythematosus (SLE), multiple sclerosis (MS), celiac disease, Type 1 diabetes mellitus, Graves' disease, inflammatory bowel disease, alopecia areata, Addison's disease, local or systemic scleroderma, psoriasis, atopic dermatitis, dermatomyositis, vitiligo, primary biliary cirrhosis, psoriatic arthritis, ankylosing spondylitis, reactive arthritis or Reiter's syndrome, rheumatoid arthritis, chronic thyroiditis (Hashimoto's thyroiditis or autoimmune thyroiditis), autoimmune hemolytic anemia, immune thrombocytopenia, Sjogren's syndrome, autoimmune retinopathy, acute anterior uveitis, myasthenia gravis, autoimmune vasculitis, antiphospholipid antibody syndrome, SAPHO syndrome, adult-onset Still's disease, and Behcet's disease.

6. The pharmaceutical composition of Claim 1, wherein the autoimmune disease is treated through the simultaneous pharmacological inhibition of oxidative stress and inflammation.

7. The pharmaceutical composition of Claim 1, wherein the autoimmune disease is treated through inhibition of oxidative stress and prostaglandin E₂ synthesis.

8. The pharmaceutical composition of Claim 1, wherein the autoimmune disease is treated through inhibition of oxidative stress and microsomal prostaglandin E synthase-1.

9. The pharmaceutical composition of Claim 1, wherein the autoimmune disease is treated through the control of T_{reg} cells.

10. The pharmaceutical composition of Claim 1, wherein the pharmaceutical composition is for humans or companion animals.

11. The pharmaceutical composition of Claim 10, wherein the companion animal is selected from the group consisting of cattle, horses, sheep, cats, chinchillas, dogs, ferrets, gerbil, pigs, and hamsters.

12. A food composition for improving symptoms of an autoimmune disease, comprising a compound represented by the following Chemical Formula (I) or a pharmaceutically acceptable salt thereof: 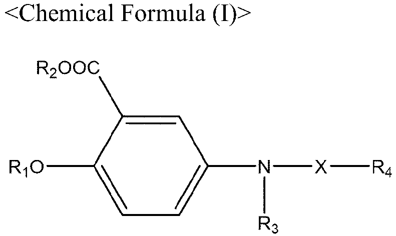 wherein,
X is selected from CO, SO₂ and (CH₂)ₙ;
R₁ is selected from hydrogen, C₁-C₆ alkyl and C₁-C₆ alkanoyl;
R₂ is hydrogen or C₁-C₆ alkyl;
R₃ is hydrogen or C₁-C₅ alkanoyl group; and
R₄ is selected from a phenyl group , a phenoxy group , and a 5- to 10-membered aryl group optionally substituted with one or more independently selected from nitro, halogen, C₁-C₆ alkyl, C₁-C₆ haloalkyl, C₁-C₅ alkoxy and C₁-C₅ haloalkoxy;
n is an integer from 1 to 5.
